# EUROPEAN PATENT APPLICATION

(11) **EP 1 209 609 A1**
(43) Date of publication of application: **29.05.2002**
(21) Application number: 01912372.8
(22) Date of filing: 15.03.2001
(51) Int. Cl.: G06F 17/60

(54) **FOOD ADVISING SYSTEM FOR DIET-RESTRICTED PERSON**

(30) Priority: 15.03.2000 JP 2000072987
(71) Applicant: Matsushita Electric Works, Ltd., Kadoma-shi, Osaka-fu 571-8686 (JP)
(72) Inventor: YOSHIDA, Keiichi, Kadoma-shi, Osaka 571-8686 (JP); ISHINO, Koichi, Kadoma-shi, Osaka 571-8686 (JP); MAEDA, Mitsuhide, Kadoma-shi, Osaka 571-8686 (JP); HEIUCHI, Takahiro, Kadoma-shi, Osaka 571-8686 (JP); HATANAKA, Tomoyuki, Kadoma-shi, Osaka 571-8686 (JP); NISHIMURA, Osamu, Kadoma-shi, Osaka 571-8686 (JP)
(74) Representative: Goddar, Heinz J., Dr.
(86) International application number: JP0102033
(87) International publication number: WO0169477

(57) **Abstract**

The meal advice system of the present invention provides appropriate next meals in consideration of the previous meal taken by a dieter. The previous meals are transmitted as an image data from a dieter terminal to a server which responds to relay the image data to an analyst terminal for requesting the analysis of foods contained in the meal. The analysis result is transmitted back to the server where nutrient constituents of the analysis result are compared with prescribed nutrient amounts needed for the dieter to select a collection of the next meals containing foods compensating the deficient nutrient amounts. Then, a proposed menu containing the collection of the meals is transmitted to the dieter and/or a meal assistant who serves the meal to the dieter.

## Description

### TECHNICAL FIELD

The present invention relates to a meal advice system for dieters, and more particularly to a system for advising proper meals meal for the dieters such as diabetic or obese people requiring limited calories.

### BACKGROUND ART

Japanese Patent Early Publication No. 7-93287, No. 8-123856, and No. 10-91584, and No. 10-295651 propose a nutrient management system utilizing a computer. The system includes a server and a remote terminal which is utilized by a user to exchange information about food eaten or intended to be eaten with the server so as to obtain nutrient analysis of the food from the server. Although the system gives the nutrient analysis of the food or the meal, the selection of a next meal have to be made still on the side of a meal administrator and is therefore cumbersome for people who is not familiar with a food management scheme referring to a food exchange group table. Thus, it has been not easy to provide an optimum food management. In addition, there has been difficulty in manipulating the remote terminal in order to enter detail data as to the kind and amount of the previous food eaten and to transmit the data to the server.

### DISCLOSURE OF THE INVENTION

The present invention has been achieved in view of the above problem and has a primary object of providing a meal advice system capable of proposing a suitable next meal in consideration of an analysis of the previous meal eaten by a dieter. The meal advice system in accordance with the present invention proposes a next meal based upon the analysis of the previous meal eaten by the dieter, and utilizes a remote terminal means capable of sending information about the previous meal. The system includes a meal advice center having a server linked to a database as well as linked through a communication network to the remote terminal means for exchanging information about the previous and next meals.

The database has at least the following tables.
- A dieter table which stores, for each of the individual dieters, an identification code, a network address, and a prescribed nutrient amount for each of Food Groups into which foods are categorized according to the nutrients.
- A meal-food table which stores the names of the meals and names of the foods contained in the meal.
- A food-nutrient table which stores the names of the foods, a group code identifying each Food Group into which the foods are classified, and a nutrient amount contained in per unit of the food.

While the sever is equipped with the following means.
- An image data receiving means which receives an image data of the previous meal transmitted from the remote terminal means.
- A meal analysis assisting means which provides the image data in addition to the food-nutrient table in order to assist analyzing the image data to determine the kinds and amounts of the foods contained in the previous meal, classifying the foods into the corresponding Food Groups, and calculating the nutrient amount for each of the food groups with reference to the food-nutrient table.
- A meal selecting means which compares the calculated nutrient amount for each Food Group with the prescribed nutrient amount from the dieter table in order to determine a deficient nutrient amount for each food group, and selects a plurality of next meals containing the foods compensating for the deficient nutrient amount for each Food Group with reference to the meal-food table and the food-nutrient table.
- A meal proposal means which proposes a menu listing the selected meal or meals received from the meal selecting means to the remote terminal means over the communication network.

In this manner, the present system is capable of analyzing the image data of the previous meal eaten by the dieter, calculating the amount of nutrient taken, comparing the nutrient amount with a prescribed nutrient amount allocated for each dieter to obtain a deficient nutrient amount, and proposing next meal or meals containing foods which compensate for the deficient nutrient amount. Thus, the meal administrator is only required to select one of the proposed meals for giving proper nutrients for good health of the individuals and assuring to keep consistent meal management, without bothering steps of calculating the nutritive value, selecting suitable foods, and then selecting the meals from the selected foods which are beyond the ken of the average layperson. Also, since the information about the previous meal can be transmitted as a photo image data to provide the details of the meal to the server, it is possible to reduce the requirement to be made on the side of the dieter. Further, since the server has the meal-analysis assisting means which provides the food-nutrient table to a meal analyst, the analyst is only required to figure out the foods from the meal image when obtaining the nutrient amount taken by the meal, thereby making it easy to analyze the nutrients from the meal immediately with high accuracy.

The meal analysis assisting means is linked through the communication network to an analyst terminal in order to provide the image data of the previous meal, meal-food table, and the food-nutrient table so that the analyst operating the terminal can analyze the previous meal and transmit the resulting meal analysis to the server for the selection of the next meals. Based upon the analysis, the meal-selecting means of the server compares the nutrient amount taken in the previous meal with the prescribed nutrient amount read out from the dieter table, thereby immediately finding out the deficient nutrient, selecting various foods compensating for the deficient nutrient with reference to the food-nutrient table, and selecting, with reference to the meal-food table, the meals containing the selected foods.

In a preferred embodiment, the food-nutrient table is configured to store amounts of nutrient constituents contained in the individual foods so as to enable the analysis of the foods with reference to the nutrient constituents. With the definition that the sum of the amounts of the nutrient constituents equals to the nutrient amount for each food, an exact nutrient analysis can be made for each Food Group.

The remote terminal means may be composed of a personal mobile terminal adapted to be carried by the dieter and a meal assistant terminal primarily adapted to be used by a meal assistant who serves the meal to the dieter. The personal mobile terminal has a camera taking the image of the previous meal and a transmitter sending the image, while the meal assistant terminal has a data receiving means which receives the proposed meals from the server. Thus, the information about the meal can be transmitted easily to streamline the meal management.

The personal mobile terminal is preferred to send a supplemental comment relating the previous meal together with the image data thereof so that the meal analysis assisting means can present the comment received at the image data receiving means to the analyst terminal, assisting the analysis of the meal thereat. The comment may be either superimposed on the photo image data or attached to the image data as a voice data.

The meal analysis assisting means is linked through the communication network to the analyst terminal to provide the image data, the meal-food table, as well as the food-nutrient table, so that the analyst operating the analyst terminal can analyze the previous meal and transmit the results of the analysis to the server for selection of the next meals.

Preferably, the server includes a meal verification means which is linked to an advisor terminal for requesting verification of the selected next meals from an advisor operating the advisor terminal. The meal verification means provides the dieter table, the analysis result, and the selected next meals to the advisor terminal for assisting the verification made by the advisor in consideration of the information about the dieter and the meal analysis, limiting the selected next meals to those verified in response to the verification from the advisor terminal. In this manner, a large number of the selected next meals can be restricted to those approved by the advisor with reference to the data of the individual dieters, assuring more considerate proposal of the meals.

The analyst terminal and the advisor terminal may be combined into a single counselor terminal so as to enable the meal analysis and the advise to be carried out at the same location efficiently by one or two specialists.

The database is preferred to include a meal history table which stores, with regard to each dieter, the names of the meals eaten by the dieter and a time stamp of the meal eaten. The meal history table is given by the meal verification means to the advisory terminal such that the specialist responsible for the meal verification can approve one or more of the selected meals as appropriate in consideration of the meal history of the dieter.

Further, the database is preferred to include a magnification table which stores a dieter code, the name of the meal eaten by the dieter, the date and time of the meal, and a magnification of the meal as determined based upon the image data thereof in relation to a standard size. The magnification table is given by the meal analysis assisting means to the analyst terminal such that the analyst can be well assisted to re-analyze the past meals by multiplying the amounts of the food contained in the past meals by that magnification.

Also, the database is preferred to include a meal history table which stores, with regard to each dieter, the names of the meals eaten by the dieter and a time stamp of the meal eaten. The meal proposal means operates to compare the next meals provided by the meal selecting means with the meals stored in the meal history table and dated within a predetermined past period, for example, the past two weeks in order to extract the meals not duplicating the meals dated in this period, and presenting the menu of thus extracted meals. Therefore, it is possible to avoid proposing similar meals.

In addition, the meal proposal means may compare the next meals provided by the meal selecting means with the meals which are stored in the meal history table and have date records within a predetermined time range around the same date of one year before, extracts the meals duplicating the meals taken in the time range, sort the extracted meals in a descending order of frequency of duplication, and present the menu of the next meals thus sorted. Therefore, it is made possible to propose the meals which matches with the season-dependent preference of the dieter.

Further, the dieter table may includes a local code indicating one of predefined regions to which the dieter belongs. With this arrangement, the meal proposal means can compare the next meals provided by the meal selecting means with the meals which are stored in the meal history table with regard to other dieters having the same local code and which have date records within a predetermined time range around the same date of one year before, extract the meals duplicating the meals occurring in the time range, sort the extracted meals in a descending order of frequency of duplication, and present the menu of the meals thus sorted. Thus, with due consideration of locality of the dieter, it is possible to propose the meals that are preferred by people living in the same locality.

Preferably, the meal proposal means is configured to, after extracting the next meals not duplicating the meals eaten in the past predetermined period, say, two weeks, compare thus extracted meals with the meals which have date records within a predetermined time range around the same date of one year before so as to give a first score to the extracted meals duplicating the meals occurring in the time range, and also compare the extracted meals with the meals for the other dieters having the same local code and date records within a predetermined time range around the same date of one year before so as to give a second score to the next meals duplicating the meals of the other dieters occurring in the time range. Thereafter, the meal proposal means counts the points given to the extracted next meals and sorts the same in a descending order of the scored points, and present the menu of the next meals thus sorted. With the use of the above scheme, it is made possible to provide more consistent proposal of the meals in consideration of the preference and the locality of the dieter, while avoiding the duplication of the meals eaten within the past short time period. In this connection, it is possible to make suitable weighting as to the order of the proposed meals, for example, by assigning different points to the first score than the second score.

The meal history table in the present system is preferred to store, with regard to each dieter, a meal type indicating that the meal is for one of the breakfast, lunch, and dinner. In this connection, the meal proposal means is configured to propose the menu listing the next meals for dinner based upon the result of the analysis which shows the sum of the nutrient amount for each Food Group with regard to the previous meals eaten as the breakfast and the lunch. Therefore, it is possible to make the meal management per one day for the dieter.

The database is preferred to include a meal image table which stores a meal code identifying the meal and a photo image of the meal. In this connection, the meal proposal means is configured to relate the selected next meal to the corresponding photo image and present the photo image together with the next meals, thereby providing more direct visual information as to the next meal to the dieter or the meal assistant.

Further, the meal analysis assisting means may be configured to prepare, based upon the analysis result of the previous meal transmitted from the analysis terminal, an analysis report demonstrating the deficient nutrient amount of each Food Group with regard to the previous meal, and to send it together with the menu of the next meals to the remote terminal means.

In this connection, the database is preferred to include an analysis table which stores a date of making the analysis in accordance with the meal analysis assisting means, a meal type indicating which one of the breakfast, lunch, and dinner corresponds to the previous meal analyzed and acknowledged by the dieter to be eaten in accordance with the menu, as well as the nutrient amount calculated for each Food Group contained in the meals analyzed. The server includes a report means which retrieves, from the analysis table, the nutrient amount for each Food Group with regard to a number of the previous meals eaten during a past predetermined time period, prepares an analysis report showing a chart of the nutrient amount for each Food Group taken during the past predetermined period, and transmits the analysis report periodically to the remote terminal means. Thus prepared analysis report helps the dieter to recognize the dietary habit of its own for a health care purpose.

Still further, the database may include a caterer table which stores names, addresses of caterers, and a list of meals available from each of the caterers. With reference to the caterer table, an agent means included in the server appoints the caterer who can serve the selected next meals, prompts the remote terminal means to request whether or not to order the next meal from the appointed caterer, and places the order to the caterer when so requested. In this manner, the dieter or the meal assistant can enjoy necessary meal service without being involved in contacting directly with the caterer.

Although the above system is preferred to include the meal analysis assisting means operating to provide the image data of the previous meals from the dieter as well as the food-nutrient table to the analyst in order to facilitate the analysis of the previous meal, the present invention should not be necessarily limited to this mode. That is, in case when the analysis as to the nutrient amount of the previous meals is available without relying upon the meal analysis assisting means, the system can successfully operate, based upon the analysis available through other than the meal analysis assisting means, to calculate the deficient amount for each Food Group, to select the foods compensating for the deficient amount, and to select the next meals containing the foods thus selected, thereby determining the next meals immediately without relying upon human power, in accordance with a concept of the present invention.

These and still other advantages and features of the present invention will become apparent from the following description of the embodiments when taken in conjunction with the attached drawings.

### BRIEF DESCRIPTION OF THE DRAWINGS

FIG. 1 is a schematic view illustrating a meal advice system in accordance with the present invention;
FIG. 2 is an explanatory view illustrating the structures of a dieter table, an image table, and an extra image table contained in a database utilized in the present system;
FIG. 3 is an explanatory view illustrating the structures of a meal-food table, a food-nutrient table, an analysis results table, and a meal magnification table contained in the database of the above system;
FIG. 4 is an explanatory view illustrating the structures of a proposed meal image table, a proposed meal table, a meal history table, and an advice table contained in the database of the above system; and

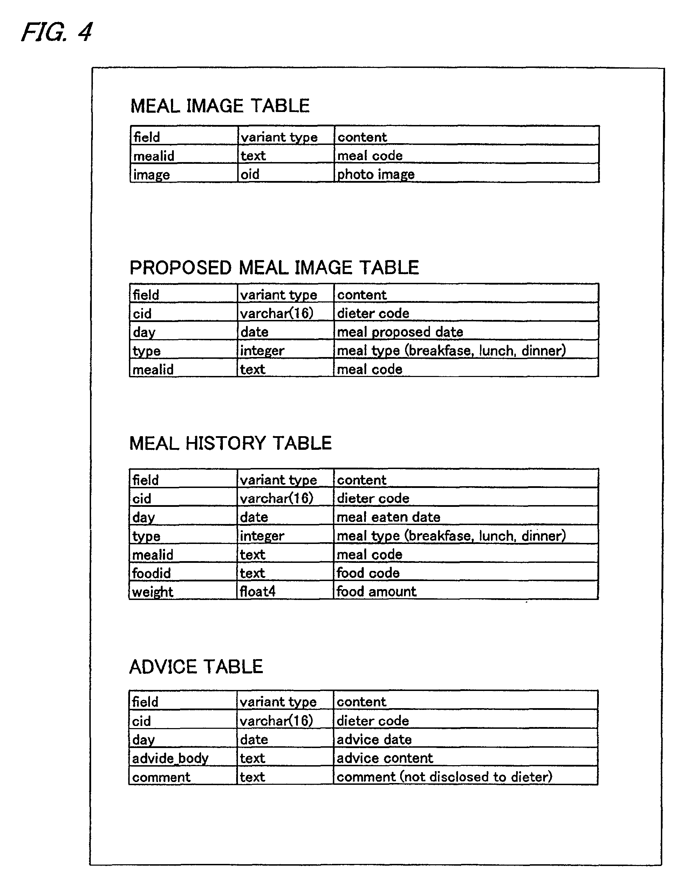

FIG. 4 is an explanatory view illustrating the structures of an analyst/advisor table, analyst/advisor schedule table, and a caterer table contained in the database of the above system.

### BEST MODE FOR CARRYING OUT THE INVENTION

The meal advice system in accordance with the present invention provides a dietary management service of analyzing one or more previous meals eaten by a dieter and proposing a menu listing a plurality of next meals selected in consideration of the analysis of the previous meals. In particular, the system is designed to operate on a daily basis to propose the meals for a dinner in consideration of the breakfast and the lunch eaten. The system is designed for providing the dietary service specifically but not limited to a diabetic and a meal assistant such as a member of the family who cares to serve the meal for the diabetic.

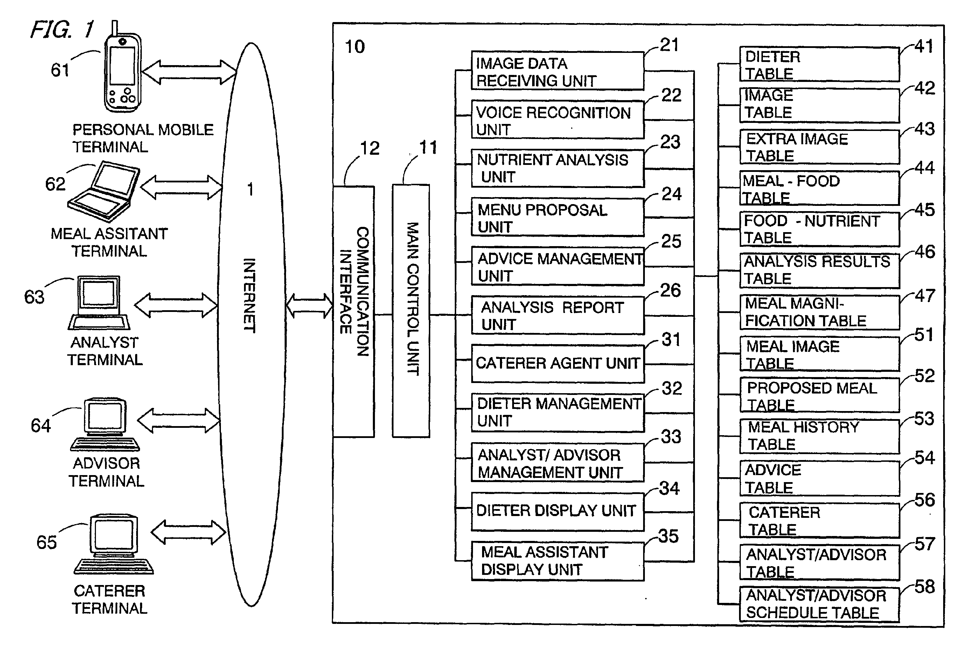

The present system is implemented by a server **10** installed in a meal advice center. As shown in FIG. 1, the server **10** is linked to a database provided inside or outside of the center and is also linked through a communication network **1** such as the Internet to various discrete terminals operated by different persons involved in the system. The discrete terminals include a personal mobile terminal **61** carried by each dieter, a meal assistant terminal **62** for use by a meal assistant, an analyst terminal **63** to be operated by a meal analyst, for example, dietitian, an advisor terminal **64** to be operated by a specialized advisor giving an advice on the details of the meal, and a caterer terminal **65** operable by a caterer servicing meals.

The personal mobile terminal **61** includes a camera taking a picture of the previous meal which is transmitted together with information related to the image to the server **10** through the communication network. The meal is analyzed based upon its image under the supervision of the server **10**. The resulting analysis of the previous meal is transmitted together with the proposed next meals to the meal assistant terminal **62**.

The database utilized in the system includes, a dieter table **41**, an image table **42**, an extra image table **43**, a meal-food table **44**, a food-nutrient table **45**, analysis results table **46**, meal magnification table **47**, a meal image table **51**, a proposed meal table **52**, a meal history table **53**, an advice table **54**, a caterer table **56**, an analyst/advisor table **57**, and an analyst/advisor schedule table **58**.

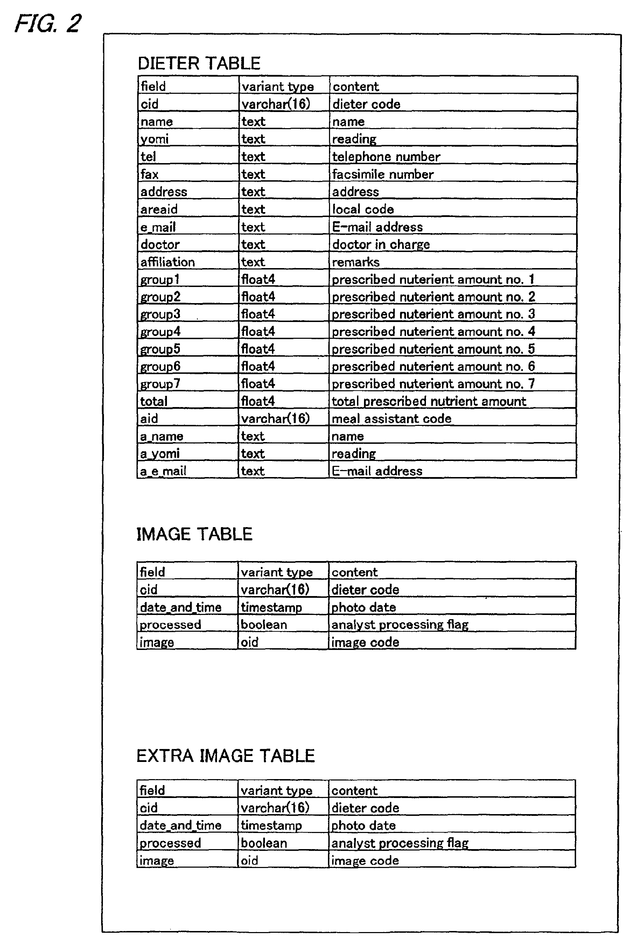

As shown in FIG. 2, the dieter table **41** has a data structure composed of various fields for entry of an identification code assigned to each of the dieters, the name, telephone number, address, E-mail address, family doctor's name, remarks, in addition to a local code identifying a locality of the dieter, and prescribed nutrient amounts (to be taken per one day) determined by diagnosis of the doctor for each of Food Groups. The Food Groups are defined in order to categorize various dairy foods based upon major ingredients or nutrients into several groups and include the following seven Food Groups.

| | |
|---|---|
| Food Group 1 | Grains |
| Food Group 2 | Fruits |
| Food Group 3 | Meats |
| Food Group 4 | Milks |
| Food Group 5 | Fats |
| Food Group 6 | Vegetables |
| Food Group 7 | Seasonings |

Each Food Group contains nutrient constituents (carbohydrate, protein, lipid, etc.) at a ratio different from each other such that one nutrient unit of the food in each Food Group can provide an estimated ratio of the nutrient constituents. The one nutrient unit is defined as an amount of the food giving, for example, 80 kirocalories energy and corresponds to different food weights for different foods. For example, "rice" in Food Group 1 has one nutrient unit equivalent to 55 g of rice, "pork meat' in Food Group 3 has one nutrient unit equivalent to 60 g of the pork meal. Such relationship is defined in a Food Exchange Table. The one nutrient unit of Food Group 1 is deemed to have a nutrient ratio of 18 g of carbohydrate, 2 g of protein, and 0 g of lipid, the one nutrient unit of Food Group 3 has a nutrient ratio of 0 g hydrocarbon, 9 g of protein, and 5 g of lipid, for example. Therefore, each food can be identified with individual nutrient constituents by the weight of that food and the Food Group in which that food is categorized, and compensation for deficient nutrient constituents can be made by identifying the Food Group and determining the dose of the nutrient unit. In the description and claims, the term "nutrient amount' is used as equivalent to one nutrient unit of the Food Group, and is defined as the sum of the individual nutrient constituents for the Food Group.

The image table **42** is configured to have fields for entry of photo image data of the meal, time stamp of the image taken, and an analyst processing flag indicating whether or not the analyst has made the analysis for the image data, in addition to the dieter code.

The extra image table **43** has the identical data structure to the image table and is used to store the image data of an extra photo image of the same meal.

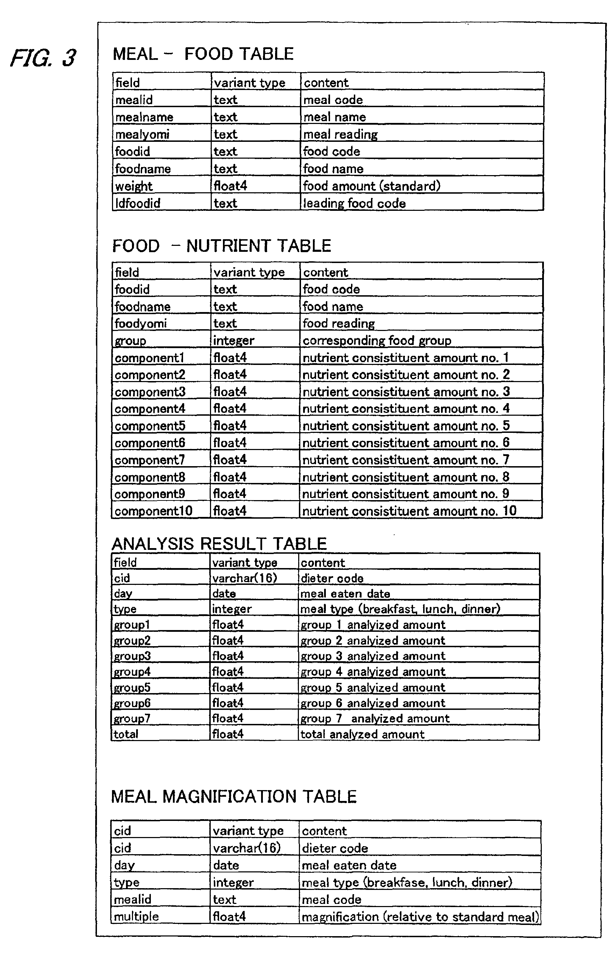

FIG. 3 shows the data structure for the meal-food table, the food-nutrient table, the analysis results table, and the meal magnification table. The meal-food table is utilized to store information about various kinds of meals, and therefore has fields for entry of a meal code identifying the kind of meal, a name of the meal, a name of food contained in that meal, a food code, a standard weight of the food, and a main food code indicating a primary food of the meal. The table is utilized for analysis of the foods contained in the meal as well as for selection of the meal based upon the foods.

The food-nutrient table has fields for entry of, in addition to a food code and food name for each food, a code of Food Group in which the food is categorized, and an amount of the nutrient constituent contained in the corresponding Food Group, and is utilized for analysis of the nutrient constituents contained in the meal.

The analysis results table is utilized to keep the results of analysis made based upon the image data provided by the individual dieters, and has fields for entry of the dieter code, date and time of taking the meal, the kind of meal indicating one of breakfast, lunch, and dinner, as well as the nutrient amounts for the individual Food Groups.

The meal magnification table stores a magnification of the meal relative to a standard size which was judged by the analyst, when analyzing the meal based upon the image data provided by the dieter in order to estimate the amounts of individual foods contained in the meal, and the nutrient amount of each of the Food Groups in which the foods are categorized. The magnification is referred to at the request of re-analyzing the meal. For this purpose, the table stores the dieter code, the date and time of the meal taken, the kind of the meal, and the meal code, in addition to the magnification.

FIG. 4 shows the data structure of the meal image table, the proposed meal image table, the meal history table, and the advice table. The meal image table stores the image of the meal proposed to the meal assistant and/or the meal administrator, and has therefore fields for entry of the meal code, and the photo image data of the meal.

The proposed meal table is used to keep information as to the next meal proposed in consideration of the analysis result, and therefore has fields for entry of the dieter code, a proposed date of the meal, the meal kinds of breakfast, lunch, or dinner, and the meal code.

The advice table is used to store the expert's advice as to the meal analysis and the proposed meal, and therefore has fields for entry of the corresponding dieter code, a date and time of the advice given, content of the advice, and a comment not disclosed to the dieter.

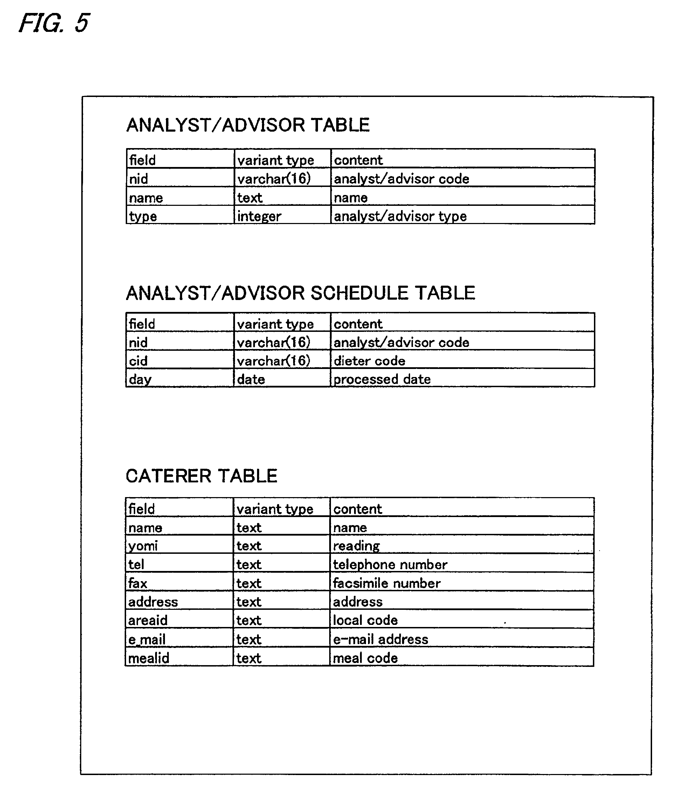

FIG. 5 shows the analyst/advisor table, the analyst/advisor schedule table, and the caterer table. The analyst/advisor table is used to record information about the analysts and the advisors registered in the present system, and include fields for entry of name and identification code of the person, as well as a type indicating which one of the analyst and the advisor.

The analyst/advisor schedule table is used to keep the record indicating whether or not the analyst has processed the analysis of the meal and whether or not the advisor has processed the advise on the meal, and includes fields for entry of identification code for the analyst or advisor, the corresponding dieter code, and a processed date and time.

The caterer table is used to make an inquiry to the terminal of the meal assistant or the dieter as to a possibility of supplying the proposed meal from the caterer, and to place the order on behalf to the caterer when so instructed, and therefore include fields for entry of a name, an identification code, an e-mail address of the caterer, as well as the meal code of the meal that the caterer can supply.

Operation of the system will be now explained. The server 10 includes a main control unit **11** programmed to implement the system and a communication interface **12** for communication with various terminals. The main control unit **11** executes a main program to govern various functional units realized respectively by module programs. The functional units include an image data receiving unit **21**, a voice recognition unit **22**, a nutrient analysis unit **23**, a menu proposal unit **24**, an advice management unit **25**, an analysis report unit **26**, a caterer agent unit **31**, a dieter management unit **32**, an analyst/advisor management unit **33**, a dieter display unit **34**, and a meal assistant display unit **35**.

The image data receiving unit **21** receives the image data of the previous meal transmitted from the personal mobile terminal **61**, then stores the image data as well as the dieter code, and the date and time of the taken image in the image table **42**, and gives "N" to the analyst processing flag field as indicating that the image has not been processed. The image table **42** is monitored by the main control unit **11** such that the main control unit **11** retrieves the data with the processing flag of "N" from the table **42** so as to locate the analyst in charge of the dieter with reference to the analyst/advisor schedule table **58**, and gives an instruction to the nutrient analysis unit **23** for issuing a request of the meal analysis to thus locate analyst. At this timing, the server **10** acknowledges that the meals corresponds to which one of breakfast, lunch, and dinner by the time stamp of the image data.

In response to the instruction, the nutrient analysis unit **23** transmits the request of making the meal analysis to the designated analyst. The server **10** is linked to the analyst terminal **63** by way of the Internet communication network **1** or a private communication line, thereby allowing the analyst terminal **63** to make an access to the dieter table **41**, image table **42**, extra image table **43**, meal-food table **44**, food-nutrient table **45**, analysis results table **46**, and meal magnification table **47**. The analyst terminal **63** executes a dedicated software to reproduce on a display the image of the meal for which the analysis is requested, for identification of the foods contained in the meal. At the same time, an indication is made to confirm that the meal corresponds to which one of breakfast, lunch, and dinner. The software gives on the display a window of a list box listing various foods such that, when the analyst clicks the listed food, for example, "rice" as being identified, an analysis box for that food appears on the display, prompting the entry of the weight of the food. In default, the analysis box gives in an amount entry-field a standard amount of the food as recorded in the meal-food table **44** and provides a magnification entry-field. The image data of the meal is designed to be taken together with a scale for measurement of the meal such that the analyst can judge the magnification of the meal relative to the standard size of the meal. Upon entry of thus judged magnification, the amount of the food is calculated as a product of the standard amount and the magnification and is displayed. The amount of the food can be corrected manually. When the amount is finally confirmed, the nutrient amount of the corresponding Food Group, in this case, Food Group 1 is displayed. The above procedure is carried out with reference to the food-nutrient table **45** through the steps of seeking a record of the food, locating the amounts of individual nutrient constituents contained in per unit weight of the food, summing up the amounts of the nutrient constituents to give the nutrient amount per unit weight of the food, and multiplying the given nutrient amount by the weight of the food to determine the nutrient amount for the Food Group in which this food is categorized. The same scheme is applied to the other foods contained in the meal for identifying the Food Group and the nutrient amount thereof. The resulting analysis of the meal is stored in the analysis results table **46** and at the same time the processing flag of the image table **42** is changed to 'Y' indicating that the analysis has been made.

Then, the nutrient analysis unit **23** of the server, after recognizing that the analysis of the previous meals of breakfast and lunch is done, is active to determine whether or not the analyzed nutrient amounts are sufficient for the prescribed nutrient amount taken per day for the dieter, and propose the dinner meal based upon the analysis of the previous meals, for example at 15:00. When the analysis of only one of the breakfast and lunch is available, no proposal of dinner meal is made. Calculation of the deficient nutrient amount is made by comparison of the analysis results recorded in the analysis results table **46** with the prescribed nutrient amount (taken per day) determined in the dieter table **41** for each Food Table with regard to the dieter concerned. When no deficiency is judged, the system provides no proposal as to the dinner meal of the same day, but informs that effect to the terminals **62** and **63** of the dieter and meal assistant.

Upon seeing the deficient nutrient amount, the menu proposal unit **24** is activated to select various foods that compensate for the deficient nutrient amount with reference to the food-nutrient table **45**, and select 10 to 15 kinds of meals that contain the selected foods with reference to the meal-food table **44**. The menu proposal unit **24** thus constitutes a meal selecting means and a meal proposal means, and operates to select the meals compensating for the deficient nutrient amounts, and subsequently to propose some of the selected meals to the dieter terminal **62** and the meal assistant terminal **63**.

With regard to a primary collection of thus selected meals, the menu proposal unit **24** checks whether or not the selected meals duplicate with meals eaten by the same dieter during a past short predetermined time period, say 14 days and provide a secondary collection of the meals excluding the duplicated meals. The meal history table **53** stores the records of the previous meals identified by analysis of the image transmitted from the dieter terminal **61** and also the records of the proposed meals confirmed to have been eaten by a response transmitted to the server from the dieter or meal assistant, together with the date of the meals taken and the meal kinds of breakfast, lunch, and dinner. By reference to the meal history table **53**, the menu proposal unit **24** can select the secondary collection of the next meals not duplicating the meals eaten during the past short time period.

Thereafter, for the purpose of taking the preference of the dieter into consideration, the meal history table **53** is referred to for comparing the meals in the second collection with the meals which were eaten within a predetermined time range around the same date of one year before, say one month before and after the same date of one year before, and gives ten (10) points to the duplicating meal or meals.

Next, the menu proposal unit **24** proceeds to reflect the food preference due to the locality of the dieter. Firstly, with reference to the dieter table **41**, ten (10) dieters of the same locality as the dieter concerned are chosen from all of the dieters. Then, the meals of the secondary collection are compared with the meals eaten by the chosen dieters during a predetermined time range around the same date of one year before, say one month before and after the same date of one year before such that the duplicating meal or meals are given one (1) point.

Thereafter, the menu proposal unit **24** operates to sort the meals of the secondary collection in a descending order of the scored points, and to determine top ten meals, followed by storing in a buffer the meal codes thereof together with the dieter code, date of proposing the meal, and the meal kind.

The server **10** responds to make the following processing in order to have an expert's advise on the menu of the determined meals. Firstly, the main control unit **11** identifies the advisor recorded in the analyst/advisor table **57** to be in charge of the dieter concerned. In this consequence, the advice management unit **25** operates to transmit an advice request for verification of the meals to the advisor terminal **64** of the identified advisor. The advice request is accompanied with the meal codes, the identification code of the dieter, date of proposing the meal, and the meal kind for the ten meals stored in the buffer, and permits an access to the dieter table **41**, image table **42**, extra image table **43**, meal-food table **44**, analysis results table **46**, meal image table **51**, proposed meal table **52**, meal history table **53**, and advice table **54**. The meal image table **51** records the image of the meals being proposed so that the advisor terminal **64** can acknowledge the proposed meals by the image thereof. The advisor terminal **64** executes a dedicated software so as to obtain from the individual tables necessary information with regard to the meals that are the subject of the advice request and give the information on the display. The information include, in addition to the prescribed nutrient amount of each Food Group for the dieter, a personal medical diagnosis stored in the remarks fields of the dieter table, and the analysis result of the proposed meals. With reference to the information, the advisor can verify three (3) meals, from the meals from the set of meals selected by the menu proposal unit **24**, as being proper to the dieter. The identification codes of thus verified meals are returned from the advisor terminal **64** to the advice management unit **25**. Whereby, the advice management unit **25** functions as a meal verification means to relay the verified meal codes to the menu proposal unit **24**. The menu proposal unit **24** responds to register, into the proposed meal table **52**, the meal codes, identification codes of the dieter, date of proposing the meals, meal kind with regard to the verified meals, and transmit the information about the meals to the terminals **63** and **62** of the meal assistant and the dieter. The information includes the names and images of the meals, and the analysis result of the previous meals so that the meal assistant or the dieter can decide the next meal with the help of the information.

The advisor terminal **64** enables the entry of the meal management advice in the form on a text to be transmitted to the dieter and the meal assistance concerned. The text is stored in the field of the advice content in the advice table **54**. The comment of the kind not to be disclosed to the dieter and the meal assistant is entered in the comment field of the advice table **54**. The advice is transmitted to the meal assistant terminal **63** and the dieter terminal **62** at the same time as the menu proposal unit **24** proposes the meals. After completion of the verification of the meals by the advisor as well as the completion of the entry of the advice and the comments, the main control unit **11** calls for the advice management unit **25** in order to enter the date of making the above processing in the processed date field of the analyst/advisor schedule table **58**.

Although the above description is directed to an exemplary case in which the analysis responsible for analyzing the previous meals is different from the advisor responsible for verifying, based upon the analysis result, a suitable number of the meals from the set of meals provided by the server, it is equally possible that the analyst can hold the advisor. In this case, the analyst terminal **63** is combined with the advisor terminal **64** into a consultant terminal that runs the software for making the above analysis as well as the advice. With this consequence, the type field of the analyst/advisor table **56** is filled with a code indicating that the analyst holds the advisor.

Further, the above description refers to the exemplary case in which the meals are proposed to the meal assistant terminal **63** and the dieter terminal **62** after the primary collection of the meals selected by the menu proposal unit **24** are processed through the steps of
1) avoiding the duplicated meals;
2) considering the preference of foods by the dieter;
3) considering the locality of the dieter; and
4) receiving the verification of the advisor.
However, the present invention should not be limited to this scheme, and may be arranged to transmit the primary collection of the meals directly to the meal assistant terminal **63** and the dieter terminal **62**, or to incorporate one or more of the above steps 1) to 4). When the steps 2) or 3) is utilized as a final processing, the meals are sorted in descending order of the duplication and presented in this order.

Still further, the present system proposes an agent service which checks whether or not the meals proposed to the meal assistant terminal **63** and the dieter terminal **62** are available from caterers and informs the availability. To this end, the server **10** includes the caterer agent unit **31** which finds out from the caterer table **56** one or more caterers that are capable of providing the caterer's service to the dieter concerned, comparing the meal codes available at the caterer with the meal codes for the next meals provided by the server **10**, and attaches to the proposed meals a comment expressing that the proposed meals can be served by the caterer, if so found. With the help of this comment, the meal assistant terminal **63** and the dieter terminal **62** can place the order to the caterer. When received the order, the caterer agent unit **31** responds to read the data from the caterer table **56** and place the order to corresponding caterer by facsimile or e-mail on behalf of the dieter for arranging the delivery of the meal to the dieter.

The server **10** includes the analysis report unit **26** which prepares an analysis report at a suitable time interval, for example, per week or month as to the analysis result stored in the analysis results table **46** as well as the content of the advice stored in the advice table **54**, and transmits it to the dieter terminal **62** and the meal assistant terminal **63**. The analysis report give a chart in the form of a table or graph showing the nutrient amount for each Food Group with regard to the meals that have been taken during the past predetermined period.

The meal assistant terminal **63** and the dieter terminal **62** execute dedicated software to prompt, at an initial window, the selection between the modes of "transmitting the previous meal", "displaying the proposed meals", and "reading analysis report" so that selection of each mode can establish the link with the server for transmitting and receiving the data relating the selected mode. The meal assistant terminal **62** is provided with a function of adding a comment by handwriting on the photo image of the meal, which comment is retained in the image table so that the analyst can analyze the meals with reference to the comment.

The comment from the dieter and the advisor may be recorded as voice data. In this case, the image table **42** and the advice table **54** are configured to have the fields for storing the voice data and the voice data. is transmitted by way of the voice recognition unit **22** provided in the server **10**.

The present system further includes the dieter management unit **32**, the analyst/advisor management unit **33**, the dieter display unit **34**, and the meal assistant display unit **35**. The dieter management unit **32** is cooperative with the dieter display unit **34** and the meal assistant display unit **35** to provide on the display of the server **10** data relating the registered particulars of the dieter and the meal assistant, while managing the dieter table **41**. The analyst/advisor management unit **33** is responsible for management of the analyst/advisor table **57** with regard to the registered particulars of the analysts and the advisors.

In the above description, the system is illustrated as being adapted for example to be implemented with the meal assistant being regarded as one of the families living with the dieter, and is therefore configured to record the information about the meal assistant and the e-mail address in the dieter table **41** . However, when the meal assistant is an independent entity having different residence from the dieter, the database may be configured to additionally include an assistant table for storing a code, name, address, telephone number, facsimile number, and e-mail address of the meal assistant, as well as the corresponding code of the dieter in order to fetch necessary data from the assistant table.

Although the above description is made to demonstrate one example for proposing the meal for a dinner as a daily service based upon the analysis of the breakfast and the lunch taken, the present invention should not be interpreted to be limited to the particular service, but may be implemented to propose the next meals to be taken several times in series based upon the previous meals taken several times.

## Claims

1. A meal advice system for dieters each equipped with remote terminal means capable of sending information about a previous meal eaten and of receiving information about a next meal to be eaten, said system comprising:
a meal advice center having a server linked to a database and linked through a communication network to said remote terminal means to exchange the information about the previous and next meals,
said database including:
a dieter table storing, for each of the individual dieters, an identification code, a network address, and a prescribed nutrient amount for each of Food Groups into which foods are categorized according to the nutrients
a meal-food table storing the names of the meals and names of foods contained in the meal;
a food-nutrient table storing the names of the foods, a group code identifying each the Food Group into which the foods are classified, and a nutrient amount contained in per unit of the food,
said server including:
image data receiving means for receiving an image data of the previous meal transmitted from the remote terminal means;
meal analysis assisting means which provides the image data in addition to the food-nutrient table in order to assist analyzing the image data to determine the kinds of the foods contained in the previous meal, classifying the foods into the corresponding Food Groups, and calculating the nutrient amount for each of the Food Groups with reference to the food-nutrient table;
meal selecting means which compares the calculated nutrient amount for each Food Group with the prescribed nutrient amount from the dieter table for determining a deficient nutrient amount for each Food Group, and selects a plurality of next meals containing the foods compensating for the deficient nutrient amount for each Food Group with reference to the meal-food table and the food-nutrient table;
meal proposal means which proposes a menu listing the selected next meal or meals received from the meal selecting means to the remote terminal means over the communication network.

2. The system as set forth in claim 1, wherein
said meal analysis assisting means is linked to an analyst terminal to provide the image data of the previous meal, the meal-food table, and the food-nutrient table so that an analyst operating said analyst terminal can analyze the previous meal and transmit the resulting meal analysis to the server for selection of the next meals.

3. The system as set forth in claim 1, wherein
said food-nutrient table stores individual amounts of nutrient constituents in relation to the foods having the nutrient constituents so that the sum of the amounts of the nutrient constituents equals to the nutrient amount for each of the foods, and
said meal analysis assisting means assists to sum up the amount of the nutrient constituents with reference to the food-nutrient table for obtaining the nutrient amount for each of the Food Groups.

4. The system as set forth in claim 1, wherein
the remote terminal means is composed of a personal mobile terminal adapted to be carried by the dieter, and a meal assistant terminal adapted to be used by a meal assistant who serves the meal to the dieter,
said personal mobile terminal having a camera for taking the image of the previous meal, and a transmitter for sending the image, and
said meal assistant terminal having a data receiving means for receiving the menu from the server and displaying the menu.

5. The system as set forth in claim 1, wherein
said image data receiving means is capable of receiving a comment by the dieter relating to the previous meal together with the image data of the previous meal, and
said meal analysis assisting means provides the comment for the analysis of the previous meal.

6. The system as set forth in claim 5, wherein
said comment is superimposed on the image data which is a photo image of the previous meal.

7. The system as set forth in claim 5, wherein
said comment is transmitted as a voice data attached to the image data which is a photo image of the previous meal.

8. The system as set forth in claim 2,
said server further includes a meal verification means which is linked to an advisor terminal for requesting verification of the selected next meals from an advisor operating the advisor terminal,
said meal verification means providing the dieter table, the meal analysis, and the selected next meals to the advisor terminal for assisting the verification in consideration of the information about the dieter and the meal analysis result, and
said meal verification means responding to the verification from the advisor terminal to limit the selected next meals to those verified.

9. The system as set forth in claim 8, wherein
said analyst terminal and said advisor terminal are combined into a single counselor terminal.

10. The system as set forth in claim 2, wherein
said database further includes a meal history table storing, with regard to each of the dieters, the names of the meals eaten by the dieter together with a date of the meal eaten,
said meal verification means providing the meal history table to the advisor terminal for assisting the verification of the meals.

11. The system as set forth in claim 2, wherein
said database includes a magnification table storing a dieter code, the name of the meal eaten by the dieter, the date of the mean eaten, and a magnification of the meal as determined based upon the image data thereof in relation to a standard size;
said meal analysis assisting means providing the magnification table to the analyst terminal in order to assist re-analyzing the past meal in such a manner as to multiply the amounts of the food contained in the past meal by the magnification.

12. The system as set forth in claim 1, wherein
said database further includes a meal history table storing, with regard to each of the dieters, the names of the meals eaten by the dieter together with a date of the meal eaten,
said meal proposal means comparing the next meals provided by the meal selecting means with the meals stored in the meal history table and having the date within a predetermined past period in order to extract the meals not duplicating the meals in the predetermined past period, and presenting the menu of thus extracted meals.

13. The system as set forth in claim 1, wherein
said database further includes a meal history table storing, with regard to each of the dieters, the names of the meals eaten by the dieter together with a date of the meal eaten,
said meal proposal means comparing the next meals provided by the meal selecting means with the meals which are stored in the meal history table and have date records within a predetermine time range around the same date of one year before, selecting the meals duplicating the meals occurring in said time range, sorting the selected meals in a descending order of frequency of the duplication, and presenting the menu of the next meals thus sorted.

14. The system as set forth in claim 1, wherein
said database further includes a meal history table storing, with regard to each of the dieters, the names of the meals eaten by the dieter together with a date of the meal eaten,
said dieter table including a local code indicating one of predefined local regions to which the dieter belongs,
said meal proposal means comparing the next meals provided by the
meal selecting means with the meals which are stored in the meal history table with regard to other dieters having the same local code and which have date records within a predetermine time range around the same date of one year before, extracting the meals duplicating the meals occurring in the time range, sorting the extacted meals in a descending order of frequency of the duplication, and presenting the menu of the next meals thus sorted.

15. The system as set forth in claim 1, wherein
said database further includes a meal history table storing, with regard to each of the dieters, the names of the meals eaten by the dieter together with a date of the meal eaten,
said dieter table including a local code indicating one of predefined local regions to which the dieter belongs,
said meal proposal means comparing the next meals provided by the meal selecting means with the meals which are stored in the meal history table with regard to the same dieter in a predetermined past period so as to extract the meals not duplicating the meals in the predetermined past period, and providing a set of thus extacted meals,
said meal proposal means comparing the set of the extracted meals with the meals which are stored in the meal history table for the same dieter and which have date records within a predetermine time range around the same date of one year before, providing a first score to the extracted meals duplicating the meals occurring in the time range,
said meal proposal means comparing the set of the extracted meals with the meals which are stored in the meal history table with regard to other dieters having the same local code and which have date records within a predetermine time range around the same date of one year before, providing a second score to the extracted meals duplicating the meals of the other dieters occurring in the time range, said second score having different points than the first score,
said meal proposal means counting the points given to the extracted meals and sorting the same in a descending order of the scored points, and presenting the menu of the next meals thus sorted.

16. The system as set forth in claim 15, wherein
said first score has a higher point than the second score.

17. The system as set forth in claim 15, wherein
said meal proposal means selects the predetermined number of the next meals having the higher points.

18. The system as set forth in claim 1, wherein
said database further includes a meal history table storing, with regard to each of the dieters, the names of the meals eaten by the dieter together with a date record and a meal type indicating that the meal is eaten on which day and as which one of the breakfast, lunch, and dinner,
said meal analysis assisting means providing the meal history table in order to assist to sum the nutrient amount for each Food Group with regard to the two immediately previous meals eaten as the breakfast and the lunch, and
said meal proposal means proposing the menu listing the next meals to be eaten as the dinner.

19. The system as set forth in claim 1, wherein
said database further includes a meal image table storing a meal code identifying the meal and a photo image of the meal,
said meal proposal means relating the selected next meals to the photo image of the corresponding meal with reference to the meal image table and presenting the photo images together with the menu of the next meals.

20. The system as set forth in claim 1, wherein
said meal analysis assisting means prepares an analysis report identifying the deficient nutrient amount of each Food Group with regard to the previous meal,
said server including a report means which provides the analysis report to the remote terminal means together with the menu of the next meals.

21. The system as set forth in claim 1, wherein
said database includes an analysis table storing a date of making the analysis in accordance with the meal analysis assisting means, a meal type indicating which one of the breakfast, lunch, and dinner corresponds to the previous meal analyzed and acknowledged to be eaten by the dieter in accordance with the menu, and the calculated nutrient amount of each Food Group for the meal,
said server including a report means which retrieves, from the analysis table, the nutrient amount of each Food Group with regard to previous meals eaten during a past predetermined time period, prepares an analysis report showing a chart of the nutrient amount of each Food Group taken during the past predetermined time period, and transmits the analysis report to the remote terminal means.

22. The system as set forth in claim 1, wherein
said database further includes a caterer table storing names and addresses of caterers, and list of meals available from each of the caterers, and
said server further including an agent means for appointing the caterer who can serve the selected next meals with reference to the caterer table and the meal-food table, prompting the remote terminal means to request whether or not to order the next meal from the appointed caterer, and placing the order to the caterer on behalf of the remote terminal means when so requested.

23. A meal advice system for dieters each equipped with a remote terminal means, said system comprising:
a meal advice center having a server linked to a database and linked through a communication network to said remote terminal means to exchange the information about a meal,
said database including:
a dieter table storing, for each of the individual dieters, an identification code, a network address, and a prescribed nutrient amount for each of Food Groups into which foods are categorized according to the nutrients
a meal-food table storing the names of the meals and names of foods contained in the meals;
a food-nutrient table storing the names of the foods, a group code identifying each Food Group into which the foods are classified, and a nutrient amount contained in per unit of the food,
said server including:
meal selecting means which compares a nutrient amount obtained by analysis of a previous meal eaten by the dieter for each Food Group, with the prescribed nutrient amount given from the dieter table for determining a deficient nutrient amount for each Food Group, and selects a plurality of next meals containing the foods compensating for the deficient nutrient amount for each Food Group with reference to the meal-food table and the food-nutrient table; and
meal proposal means which proposes a menu listing the selected next meal or meals received from the meal selecting means to the remote terminal means over the communication network.
